# EUROPÄISCHE PATENTSCHRIFT

(11) **EP 0 423 280 B1**
(45) Veröffentlichungstag und Bekanntmachung des Hinweises auf die Patenterteilung: **23.08.1995**
(21) Anmeldenummer: 90906178.0
(22) Anmeldetag: 18.04.1990
(51) Int. Cl.: A61B 17/68

(54) **FIXIERUNGSSYSTEM FÜR RÖHRENKNOCHENFRAKTUREN**
FIXATION SYSTEM FOR TUBULAR BONE FRACTURES
SYSTEME DE FIXATION POUR FRACTURES D'OS TUBULAIRES

(30) Priorität: 18.04.1989 DE 3912703
(43) Veröffentlichungstag der Anmeldung: 24.04.1991
(73) Patentinhaber: Baumgart, Rainer, Dipl.-Ing. Dr. med., D-81479 München (DE); BETZ, Augustin, D-82319 Starnberg (DE); SEIBOLD, Reiner, D-85410 Haag (DE)
(72) Erfinder: BAUMGART, Rainer, D-81479 München 90 (DE)
(74) Vertreter: von Füner, Alexander, Dr.
(86) Internationale Anmeldenummer: EP9000622
(87) Internationale Veröffentlichungsnummer: WO9012547

(56) Entgegenhaltungen:
- EP-A- 0 177 270
- EP-A- 0 240 034
- FR-B- 742 618
- GB-A- 2 075 346
- US-A- 4 456 004
- US-A- 4 794 918

## Beschreibung

Die Erfindung betrifft eine Fixierungsvorrichtung für Röhrenknochenfrakturen nach dem Oberbegriff des Patentanspruchs 1.

Die Fixierungsvorrichtung wird vor allem bei Frakturen der langen Röhrenknochen der unteren Extremitäten verwendet.

Zur operativen Stabilisierung von Röhrenknochenfrakturen verwendet man bisher die Plattenosteosynthese, die Marknagelung oder einen externen Fixateur. Die Wahl des Stabilisierungsverfahrens hängt zum einen von der speziellen Bruchform ab und wird zum andern entscheidend davon bestimmt, welche Bedeutung der Operateur den biomechanischen und den biologischen Vorgängen während der Knochenbruchheilung zumißt.

Die Knochenbruchheilung wird begünstigt, wenn zwischen den beiden frakturierten Knochenstücken keine Relativbewegung stattfindet. Diese Relativbewegungen zu minimieren ist das Ziel jeder konservativen und operativen Maßnahme.

Untersuchungen in neuerer Zeit deuten jedoch darauf hin, daß diese absolute Ruhigstellung nicht in allen Phasen der Frakturheilung für alle Bewegungsrichtungen gleichermaßen erforderlich ist und im Gegenteil eine Mikrobewegung in axialer Richtung der Knochenbruchheilung sogar förderlich sein könnte. Stabilisierungssysteme wie der Marknagel und auch der Fixateur externe lassen unter bestimmten Voraussetzungen eine axiale Kompression auf die frakturierten Knochenenden bzw. den sich bildenden Kallus, bedingt durch das Körpergewicht, zu. Diese Art von Frakturheilungsförderung kann nach einer Initialphase immer dann zur Anwendung kommen, wenn entweder durch die unkomplizierte Bruchform oder durch das angewandte Fixierungssystem keine Scher- und Torsionskräfte im Bruchspalt zu erwarten sind.

Die biologischen Aspekte der Knochenbruchheilung betreffen vor allem die Gefäßversorgung in dem sich neu bildenden Knochen. Hier spielt vor allem die Knochenhaut, die den gesunden Knochen von außen umgibt, aber auch der Markraum eine entscheidende Rolle.

Für ein Fixierungssystem im Bereich der Röhrenknochen lassen sich daher folgende Anforderungen zusammenfassen, nämlich ein Ausschalten von Querkräften sowie von Biege- und Torsionsmomenten im Frakturbereich, eine bedarfsweise dosierte Normalkraft im Frakturbereich und keine zusätzlichen Blutzirkulationsstörungen durch das Fixierungssystem im Frakturbereich. Die eingangs genannten Fixierungssysteme werden diesen Anforderungen jeweils nur zum Teil gerecht.

Die Plattenosteosynthese, bei der eine schalenförmige Mehrlochplatte der Knochenhaut über eine längere Strecke, vor allem aber auch im Bereich des Frakturspaltes, anliegt, kann aufgrund von Zirkulationsstörungen zu Heilungsverzögerungen und auch zu Knochennekrosen führen.

GB - A - 2 075 346 beschreibt eine Knochenplatte, deren Querschnitt im mittleren Abschnitt verringert ist. US-A-4 974 918 bezieht sich auf eine Knochenplatte mit Mitteln zum Festlegen der Richtung der Knochenschraube bezüglich der Platte.

Die Marknagelung bzw. die Verriegelungsnagelung, bei der ein markraumfüllender Metallstab in das Innere des Röhrenknochens vorgetrieben wird, kann ebenfalls zu erheblichen Durchblutungsstörungen, hierbei jedoch von der Innenseite des Röhrenknochens, führen. Eine zentrale Schienung in der Nähe der neutralen Faser ist darüber hinaus kein guter Schutz vor Biegewechselbelastungen im Frakturbereich.

Bei dem Fixateur externe handelt es sich um ein Stabilisierungssystem, bei dem Schrauben proximal und distal des Frakturbereiches in den Knochen verankert werden und durch die Haut nach außen treten, wo sie mit einem stabilen Kraftträger verbunden werden. Ein derartiges Fixierungssystem steht nicht in räumlicher Nähe mit dem Frakturspalt und führt deshalb nicht zu Zirkulationsstörungen. Ein entscheidender Nachteil besteht jedoch darin, daß die Kraftübertragung über die langen und damit in alle Richtungen elastischen Knochenschrauben zu dem Kraftträger erfolgt und damit der Kraftverlauf über das Fixierungssystem bzw. über den Knochen schwer abzuschätzen ist. Eine bedarfsgerechte Dynamisierung, d. h. Zulassung von Normalkräften in einer späteren Phase der Knochenbruchheilung ist meist nur bedingt möglich, da aufgrund des großen Abstandes zwischen Knochenachse und Kraftträger und der damit verbundenen Biegemomente im Kraftträger meist ein Verkanten des Systems auftritt. Darüber hinaus bedeutet ein externes Stabilisierungssystem eine erhebliche Infektionsgefahr und bedarfeiner intensiven Pflege.

Ein bekanntes Stabilisierungssystem für Frakturen im Bereich der Wirbelkörper ermöglicht aufgrund seiner kompakten Bauart die Implantation unter die Haut, so daß das Infektionsrisiko verringert und der Komfort für den Patienten verbessert ist. Das starre System ist jedoch bauartbedingt nicht für die Stabilisierung von Röhrenknochenfrakturen geeignet und bedeutet hinsichtlich der Dynamisierungsmöglichkeiten lediglich eine graduelle Verbesserung gegenüber dem Fixateur externe.

Die der Erfindung zugrunde liegende Aufgabe besteht darin, die gattungsgemäße Fixierungsvorrichtung so auszubilden, daß sie unter der Haut implantierbar ist, Querkräfte sowie Biege- und Torsionsmomente im Frakturbereich ausschaltet, eine Dosierung der Normalkraft im Frakturbereich ermöglicht und Zirkulationsstörungen im Frakturbereich auf ein Minimum reduziert.

Diese Aufgabe wird ausgehend von der gattungsgemäßen Fixierungsvorrichtung mit den Kennzeichnenden Merkmalen des Patentanspruchs 1 gelöst.

Die erfindungsgemäße Fixierungsvorrichtung hat einen abgeflachten Kraftträger aus Implantatstahl, der in der Nähe des frakturierten Röhrenknochens angeordnet wird, ohne daß eine flächenhafte Berührung des Röhrenknochens stattfindet. Zwischen den beiden steifen Endabschnitten dieses Kraftträgers bzw. Fixierungselements befindet sich ein zentrales Verbindungsteil, das verglichen mit den Endabschnitten eine bestimmte Elastizität aufweist. Die Endabschnitte dienen zur Aufnahme von jeweils mindestens zwei Knochenschrauben, die in genügend großem Abstand von der Frakturzone in den stabilen Mantelschichten des Röhrenknochens festen Halt finden. Das gesamte Stabilisierungssystem ist äußerst kompakt gebaut und ermöglicht die Implantation unter die Haut.

Die Fixierungsvorrichtung läßt sich als Baukastensystem verwirklichen. Die Knochenschrauben sowie die Befestigung der Knochenschrauben im Kraftträger ist dabei standardisiert. Das den Kraftträger bildende Fixierungselement selbst ist aus einem Stück gefertigt, weist ebenfalls standardisierte Endabschnitte auf, während der zentrale Verbindungsabschnitt in verschiedenen Stärken ausgeführt werden kann, so daß der Operateur je nach Körpergewicht des Patienten und nach Frakturbefund den optimalen Kraftträger auswählen kann.

Bei der erfindungsgemäßen Fixierungsvorrichtung ist die von zwei schmalen Seiten und zwei breiten Seiten des Verbindungsabschnitts des Fixierungselements begrenzte Querschnittsfläche durch - bezogen auf die Endabschnitte - Reduzierung der Abmessungen der schmalen Seiten oder der Abmessungen der breiten Seiten oder der Abmessungen der schmalen und breiten Seiten verkleinert. Dabei beträgt die Reduzierung der Abmessungen der schmalen Seiten ebenso wie die Reduzierung der Abmessungen der breiten Seiten des Verbindungsabschnitts zweckmäßigerweise 30 - 70 %.

Die Querschnittsreduzierung in Richtung der schmalen Seite des Verbindungsabschnitts führt dazu, daß in Abhängigkeit von der Belastung des Knochens Normalkräfte im Frakturbereich auftreten. Die vom Fixierungselement abgewandte Knochenseite unterliegt dabei einer größeren Kompression als die dem Fixierungselement zugewandte Knochenseite. Nach Fortfall der Belastungskraft wirkt eine Zugkraft in umgekehrter Weise aufgrund der elastischen Rückstelltendenz des Kraftträgers. Bei wechselnder Normalkraftbelastung, beispielsweise beim Gehen, findet im Frakturspalt nahezu axial eine Zug- und Druckbelastung statt. Die Steifigkeit des Kraftträgers und der kurzen Knochenschrauben bewirkt eine ausreichende Fixierung in allen übrigen Bewegungsrichtungen, so daß keine Querkräfte oder Biegen und Torsionsmomente im Frakturspalt auftreten.

Die Einschnürung des Verbindungsabschnitts in Richtung der breiten Seite ermöglicht eine gewebeschonende Anordnung mit möglichst wenig Raum für das Fixierungselement im Bereich der Frakturzone, um keine zusätzlichen Zirkulationsstörungen hervorzurufen.

Das Fixierungselement kann aus zwei plan aufeinanderliegenden Fixierungselementteilen bestehen, die in den Endabschnitten aneinander festgelegt sind und deren Verbindungsabschnitte durch eine Spreizeinrichtung voneinander entfernbar sind. Die Spreizeinrichtung kann eine in eine Gewindebohrung im Verbindungsabschnitt des einen Fixierungselementteils eingeschraubte Madenschraube sein. Die beiden Fixierungselementteile können dabei wenigstens an ihren Verbindungsabschnitten in Richtung ihrer schmalen Seite unterschiedlich dick sein.

Bei dieser Ausgestaltung ist der das Fixierungselement bildende Kraftträger in Richtung seiner schmalen Seite geteilt, beispielsweise in einen dickeren und einen dünneren Fixierungselementteil, die plan aufeinander liegen und lediglich im Bereich der Endabschnitte miteinander fest verbunden sind, beispielsweise durch Verschrauben. Im Bereich des Verbindungsabschnitts können die beiden Fixierungselementteile voneinander weggespreizt werden.

Wenn dabei das dünnere Fixierungselementteil knochennah angeordnet ist, kann durch Aufspreizung im Bereich des Verbindungsabschnitts eine Kompression auf den Frakturspalt ausgeübt werden. Ist hingegen der dickere Fixierungselementteil knochennah angeordnet, werden die beiden Frakturenden durch Aufspreizung im Bereich des Verbindungsabschnitts voneinander entfernt, was gegebenenfalls zum Distanzerhalt bei Vorliegen einer Trümmerzone erforderlich sein kann. In beiden Fällen bleibt die elastische Komponente des Verbindungsabschnitts erhalten, bei dem zweigeteilten Kraftträger wird lediglich die Biegecharakteristik verändert.

Wenn die Knochenschrauben verschwenkbar in den Endabschnitten des Fixierungselements sitzen, ist eine bessere Anpassung an die jeweilige Fraktursituation und eine Korrektur bei Fehlbohrungen möglich. Ferner lassen sich die Knochenschrauben unter Vorspannung montieren, was unter Umständen einer Lockerungstendenz vorbeugt.

An den Endabschnitten des Fixierungselements können Führungsabschnitte für die Schäfte der Knochenschrauben vorgesehen werden. Die Knochenschrauben sind in den Fixierungselementen durch eine Spannvorrichtung gegen axiale Verschiebung gesichert und werden durch einen kegelsegmentartig ausgebildeten Führungsabschnitt bis unmittelbar zum Knocheneintritt stabilisiert.

Die erfindungsgemäße Fixierungsvorrichtung wird folgendermaßen eingesetzt:
Nach Reposition der Fraktur wird der vom Fixierungselement gebildete Kraftträger mit den beiden Knochenenden verbunden, indem zunächst mit Hilfe einer auf den Kraftträger aufgeschraubten Bohrschablone die Verankerungslöcher in die Knochencorticalis gebohrt werden und falls erforderlich anschließend ein entsprechendes Gewinde geschnitten wird. Die Knochenschraube wird dann an ihrem hinteren Ende mit einem Spannknebel gefaßt und durch den Kraftträger in den Knochen gedreht, bis sie in den beiden gegenüberliegenden Corticalisschichten festen Halt gefunden hat. Dieser Vorgang wiederholt sich an den übrigen Befestigungspunkten des Kraftträgers. Die Knochenschrauben werden nun mit einem Bolzenschneider in Höhe der Außenfläche des Kraftträgers gekürzt. Die axiale Fixierung der Knochenschrauben erfolgt mittels eines längsgeschlitzten Konus, der über die Knochenschraube geschoben wird und, angepreßt durch eine mit einem flachen Kopf versehene Schraube, die in der Mitte das abgeschnittene Ende der Knochenschraube aufnimmt, seinen festen Halt in einem ebenfalls konischen Gegenlager im Bereich der kegelsegmentartigen Führung findet.

Ist der Kraftträger in geteilter Form ausgeführt, so läßt sich mit Hilfe der von der Imbus-Madenschraube gebildeten Spreizvorrichtung nach Montage der Fixierungsvorrichtung eine Be- oder Entlastung der Frakturzone vornehmen. Durch eine kleine Hautinzision ist die Madenschraube auch im späteren Verlauf der Frakturheilung erreichbar, um die Elastizität des Kraftträgers an die jeweiligen Bedürfnisse der einzelnen Frakturheilungsstadien anzupassen.

Anhand von Zeichnungen werden Ausführungsbeispiele der Erfindung näher erläutert. Es zeigt:
- Fig. 1: perspektivisch eine Fixierungsvorrichtung, angebracht über dem Bruch eines Röhrenknochens,
- Fig. 2: die Fixierungsvorrichtung von Fig. 1 um etwa 90° gedreht.
- Fig. 3: im Schnitt die formschlüssige Befestigung der Knochenschraube im Fixierungselement,
- Fig. 4: im Schnitt die schwenkbare Befestigung der Knochenschraube im Fixierungselement um ca. 20° geschwenkt.
- Fig. 5: perspektivisch einen Endbereich eines Fixierungselements mit Röhrenknochen zur Erläuterung der Verschwenkmöglichkeiten.
- Fig. 6: in einer Ansicht wie Fig. 1 eine Modifizierung der Fixierungsvorrichtung,
- Fig. 7: im Schnitt den Verbindungsabschnitt als Einzelheit A von Fig. 6 im unverspreizten Zustand,
- Fig. 8: in einer Ansicht wie Fig. 7 den Verbindungsabschitt im gespreizten Zustand.

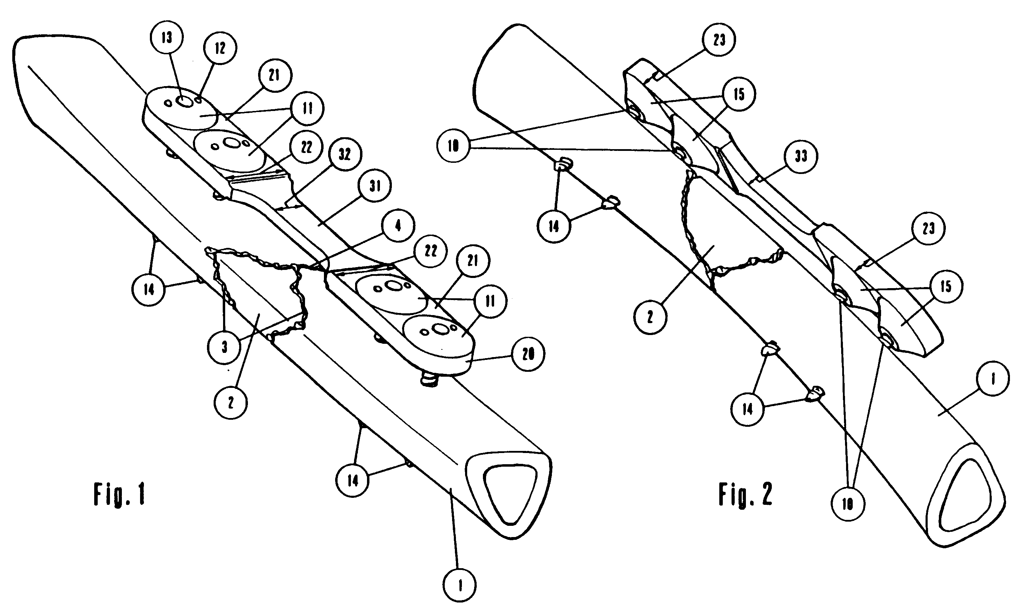

Der in Fig. 1 und 2 gezeigte Röhrenknochenabschnitt 1 ist längs eines ersten Frakturspaltes 4 gebrochen, der sich in zwei Frakturspalte 3 aufzweigt, wodurch ein herausgesprengtes Segment 2 gebildet wird, das in den Röhrenknochenabschnitt 1 wieder eingesetzt ist, wobei der Bruch als Ganzes mit Hilfe eines Fixierungselements 20 fixiert ist.

Das Fixierungselement 20 hat zwei Endabschnitte 21, die durch einen Verbindungsabschnitt 31 miteinander verbunden sind. In jedem Endabschnitt 21 sind im Abstand zwei Bohrungen vorgesehen, durch die Knochenschrauben 10 hindurchgeführt sind. Jede Knochenschraube 10 hat einen Schaft 13 und ein Gewinde 14. Der Schaft 13 jeder Knochenschraube ist in einem kegelsegmentartigen Führungsabschnitt 15 geführt, der an dem Fixierungselement 20 auf der dem Röhrenknochenabschnitt 1 gegenüberliegenden Seite angebracht ist. Das proximale Ende der Knochenschaube ist von einer, mit einem flachen Kopf und einer zentralen Bohrung versehenen Schraube 11 umgeben, die zwei diametral angeordnete Bohrungen 12 für den Eingriff der Enden eines entsprechend ausgebildeten Schraubendrehers aufweist.

Das Fixierungselement 20 hat eine langgestreckte abgeflachte Form mit im wesentlichen rechteckigem Querschnitt. Die Endabschnitte 21 sind relativ steif. Ihr Querschnitt hat zwei gegenüberliegende breite Seiten 22 und zwei gegenüberliegende schmale Seiten 23. Der Verbindungsabschnitt 31 ist verglichen mit der Steifigkeit der Endabschnitte 21 elastisch, was dadurch erreicht wird, daß seine breite Seite 32 kleiner ist als die breite Seite 22 des Endabschnitts 21 und daß seine schmale Seite 33 eine geringere Erstreckung hat als die schmale Seite 23 des Endabschnitts 21. Bei dem in den Fig. 1 und 2 gezeigten Ausführungsbeispiel ist sowohl die breite Seite 32 als auch die schmale Seite 33 des Verbindungsabschnitts 31 in ihrer Erstreckung gegenüber der breiten Seite 22 und der schmalen Seite 23 eines jeden Endabschnitts 21 verringert, nämlich um etwa 40%.

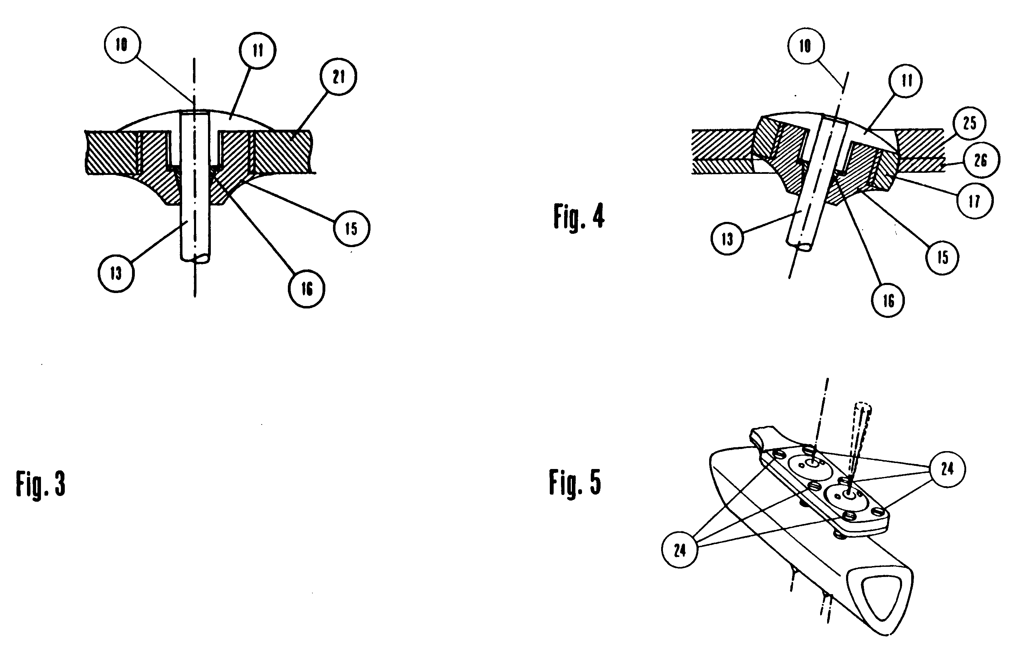

Wie in Fig. 3 gezeigt ist, sitzen die Knochenschrauben 10 in dem Endabschnitt 21 derart, daß ihr Schaft 13 sich durch einen kegelsegmentförmigen Führungsabschnitt 15 erstreckt, wobei die axiale Fixierung mit Hilfe eines längsgeschlitzten Konus 16 erfolgt, der durch die Schraube 11 in ein Gegenlager im kegelsegmentförmigen Führungsabschnitt 15 gepreßt wird. Der Führungsabschnitt 15 liegt mit seiner Basis großflächig an dem Endabschnitt 21 an und ist mit diesem kraftschlüssig verbunden.

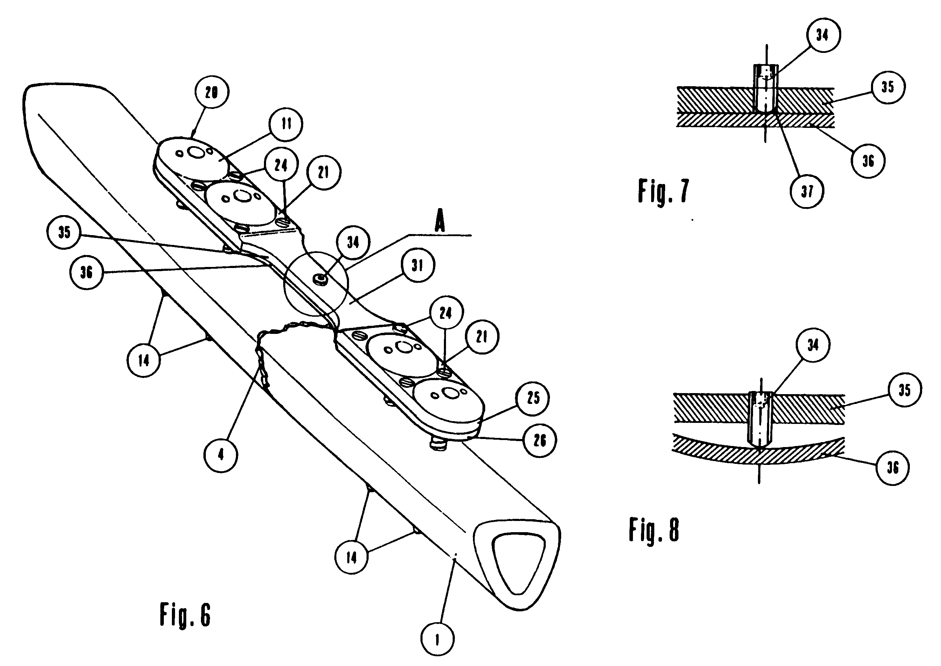

Bei der in Fig. 4 gezeigten Modifizierung ist die Knochenschraube 11 mit ihrem Schaft 13, und mit dem Führungsabschnitt 15 mit dem Konus 16 in einem Kugelabschnitt 17 gehalten, der verschwenkbar in dem bei dieser Ausführung von zwei Fixierungselementteilen 25 und 26 gebildeten Fixierungselement 20 sitzt. Durch die kraftschlüssige Fixierung des Kugelabschnitts 17 zwischen den Fixierungselementteilen 25 und 26, die aneinander durch Schrauben 24 fixiert sind, was in Fig. 5 gezeigt ist, läßt sich die Knochenschraube 10 um einen bestimmten Betrag verschwenken und somit in eine Vorbohrung im Röhrenknochenabschnitt auch dann einschrauben, wenn diese Vorbohrung nicht ganz parallel zur Vorbohrung für die andere Knochenschraube des gleichen Endabschnitts ist.

Bei der in den Fig. 6 bis 8 gezeigten Modifizierung hat der Röhrenknochenabschnitt 1 einen durchgehenden Frakturspalt 4. Das den Kraftträger bildende Fixierungselement 20 ist in der Erstreckung seiner Schmalseite in zwei Fixierungselementteile 25 und 26 unterteilt, die plan aufeinanderliegen, so daß sich die Trennfuge auch durch den Verbindungsabschnitt 31 erstreckt, der dadurch in einen Verbindungsabschnitt 35, der in den Fig. 6 bis 8 dick eingezeichnet ist, und in einen Verbindungsabschnitt 36, der dünner eingezeichnet ist, unterteilt ist. Die beiden Fixierungselementteile 25 und 26 sind im Bereich ihrer Endabschnitte 21 jeweils durch vier Schrauben 24 aufeinander fixiert. In dem dickeren, vom Röhrenknochenabschnitt 1 abgewandten Verbindungsabschnitt 35 ist in eine Gewindebohrung eine Imbus-Madenschraube 34 eingeschraubt. Mit Hilfe dieser Imbus-Madenschraube 34 läßt sich der dem Röhrenknochenabschnitt 1 zugewandte Verbindungsabschnitt 36 aus der in Fig. 7 gezeigten plan anliegenden Stellung in die in Fig. 8 gezeigte nach außen gewölbte Stellung bringen, wodurch die am Röhrenknochenabschnitt 1 beidseitig des Frakturspalts 4 wirksam werdenden Kräfte verändert werden können.

## Patentansprüche

1. Fixierungsvorrichtung für Röhrenknochenfrakturen (3, 4) mit einem langgestreckten, abgeflachten Fixierungselement (20),
- das aus einem gewebekompatiblen hochfesten Material, wie Implantatstahl, besteht,
- dessen Querschnitt im wesentlichen rechteckig ist,
- das steife Endabschnitte (21) mit jeweils mindestens zwei sich durch seine gegenüberliegenden breiten Seiten erstreckende Bohrungen für die Durchführung von Knochenschrauben (10) aufweist, die einen Schaft (13) haben, und
- zwischen dessen steifen Endabschnitten (21) sich ein elastischer Verbindungsabschnitt (31) mit einem Materialquerschnitt erstreckt, der kleiner ist als der der Endabschnitte (21),
dadurch gekennzeichnet,
- daß in den Bohrungen der Endabschnitte (21) des Fixierungselements (20) Einspannvorrichtungen (11, 15, 16) vorgesehen sind, die die Knochenschrauben (10) in dem Fixierungselement (20) axial fixieren und bis unmittelbar zum Knocheneintritt stabilisieren, so daß das Fixierungselement (20) in der Nähe des frakturierten Röhrenknochens angeordnet werden kann, ohne daß eine flächenhafte Berührung mit dem Knochen stattfindet, eine Implantation unter die Haut jedoch möglich ist.

2. Fixierungsvorrichtung nach Anspruch 1, dadurch gekennzeichnet, daß jede Einspannvorrichtung
- in jeder Bohrung der Endabschnitte (21) einen Führungsabschnitt (15) für den Schaft (13) der Knochenschraube (10) mit einem konischen Gegenlager,
- einen längsgeschlitzten, über den Schaft (13) der Knochenschraube (10) schiebbaren Konus (16) und
- einen flachen Kopf (11) mit einer Bohrung zum Pressen des längsgeschlitzten Konus (16) in das konische Gegenlager aufweist.

3. Fixierungsvorrichtung nach Anspruch 1 oder 2, dadurch gekennzeichnet, daß der Führungsabschnitt (15) mit seiner Basis großflächig an dem Endabschnitt (21) anliegt und mit diesem kraftschlüssig verbunden ist.

4. Fixierungsvorrichtung nach Anspruch 1 oder 2, dadurch gekennzeichnet, daß der Führungsabschnitt (15) in einem Kugelabschnitt (17) gehalten ist, der verschwenkbar in einer Bohrung im Endabschnitt (21) kraftschlüssig sitzt.

5. Fixierungsvorrichtung nach einem der vorhergehenden Ansprüche, dadurch gekennzeichnet, daß die von zwei schmalen Seiten und zwei breiten Seiten des Verbindungsabschnitts (31) des Fixierungselements (20) begrenzte Querschnittsfläche durch - bezogen auf die Endabschnitte (21) - Reduzierung des Abmessungen (33) der schmalen Seiten oder der Abmessungen (32) der breiten Seiten oder der Abmessungen (33, 32) der schmalen und der breiten Seiten verkleinert ist.

6. Fixierungsvorrichtung nach Anspruch 5, dadurch gekennzeichnet, daß die Reduzierung der Abmessungen (33) der schmalen Seiten des Verbindungsabschnitts (31) oder daß die Reduzierung der Abmessungen (32) der breiten Seiten des Verbindungsabschnitts (31) 30 % bis 70 % beträgt.

7. Fixierungsvorrichtung nach einem der vorhergehenden Ansprüche, dadurch gekennzeichnet, daß das Fixierungselement (20) aus zwei plan aufeinanderliegenden Fixierungselementteilen (25, 26) besteht, die in den Endabschnitten (21) aneinander festgelegt (24) sind.

8. Fixierungsvorrichtung nach Anspruch 7, dadurch gekennzeichnet, daß die Verbindungsabschnitte (35, 36) der Fixierungselementteile (25, 26) durch eine Spreizeinrichtung (34, 37) voneinander entfernbar sind.

9. Fixierungsvorrichtung nach Anspruch 8, dadurch gekennzeichnet, daß die Spreizeinrichtung eine in eine Gewindebohrung (37) im Verbindungsabschnitt (35) des einen Fixierungselementteils (25) eingeschraubte Madenschraube (34) ist.

10. Fixierungsvorrichtung nach einem der Ansprüche 7 bis 9, dadurch gekennzeichnet, daß die beiden Fixierungselementteile (25, 26) wenigstens an ihren Verbindungsabschnitten (35, 36) in Richtung ihrer schmalen Seite unterschiedlich dick sind.

## Claims

1. Fixation device for fractures (3, 4) of tubular bones, having an elongated flattened fixation element (20)
- which comprises a tissue-compatible high-strength material, such as implant steel;
- the cross-section of which is substantially rectangular;
- which has rigid end portions (21) each of which has at least two drilled holes extending through the opposing wide faces of the fixation element for the passage of bone screws (10) which have a shank (13); and
- between the rigid end portions (21) of which extends a resilient connecting portion (31) having a material cross-section which is smaller than that of the end portions (21);
characterised in that
- clamping devices (11, 15, 16) are provided in the drilled holes of the end portions (21) of the fixation element (20) and fix the bone screws (10) axially in the fixation element (20) and stabilise them to immediately up to the entrance to the bone so that the fixation element (20) can be arranged in the vicinity of the fractured tubular bone without surface contact with the bone,
implantation under the skin, however, being possible.

2. Fixation device according to Claim 1, characterised in that each clamping device has
- in each drilled hole of the end portions (21), a guide portion (15) for the shank (13) of the bone screw (10) with a conical abutment;
- a longitudinally slotted cone (16) which can be slipped over the shank (13) of the bone screw (10); and
- a flat head (11), having a drilled hole, for pressing the longitudinally slotted cone (16) into the conical abutment.

3. Fixation device according to Claim 1 or 2, characterised in that the guide portion (15) lies with the large surface of its base on the end portion (21) and is connected thereto in form-fitting manner.

4. Fixation device according to Claim 1 or 2, characterised in that the guide portion (15) is held in a spherical portion (17) which sits pivotably and in form-fitting manner in a drilled hole in the end portion (21).

5. Fixation device according to any one of the preceding claims, characterised in that the cross-sectional area delimited by two narrow faces and two wide faces of the connecting portion (31) of the fixation element (20) is reduced by reducing, relative to the end portions (21), the dimensions (33) of the narrow faces or the dimensions (32) of the wide faces or the dimensions (33, 32) of the narrow and wide faces.

6. Fixation device according to Claim 5, characterised in that the reduction in the dimensions (33) of the narrow faces of the connecting portion (31) or the reduction in the dimensions (32) of the wide faces of the connecting portion (31) is 30% to 70%.

7. Fixation device according to any one of the preceding claims, characterised in that the fixation element (20) comprises two fixation element components (25, 26) which lie flat one on top of the other and are fastened (24) against one another in the end portions (21).

8. Fixation device according to Claim 7, characterised in that the connecting portions (35, 36) of the fixation element components (25, 26) can be moved apart from one another by a spreading device (34, 37).

9. Fixation device according to Claim 8, characterised in that the spreading device is a grub screw (34) screwed into a threaded drilled hole (37) in the connecting portion (35) of the first fixation element component (25).

10. Fixation device according to any one of Claims 7 to 9, characterised in that the two fixation element components (25, 26) have different thicknesses, at least at their connecting portions (35, 36), in the direction of their narrow face.

## Revendications

1. Système de fixation pour fractures d'os tubulaires (3,4) avec un élément de fixation allongé aplati (20),
- en matériau résistant histocompatible, comme l'acier d'implant,
- dont la section est pour l'essentiel rectangulaire,
- qui présente des sections finales rigides (21) avec à chaque fois au moins deux alésages s'étendant dans ses côtés larges opposés, prévus pour le passage des vis à os (10) comportant une tige (13)
et
- entre les sections finales rigides (21) duquel s'étend une partie de liaison élastique (31) avec une section de matériau inférieure à celle des sections finales (21)
caractérisé en ce que dans les alésages des sections finales (21) de l'élément de fixation (20) sont prévus des dispositifs de serrage (11, 15, 16) qui fixent axialement les vis à os (10) dans l'élément de fixation (10) et les stabilisent directement jusqu'à l'entrée dans l'os de sorte que l'élément de fixation (20) peut être monté à proximité de l'os tubulaire fracturé sans qu'un contact superficiel n'ait lieu avec l'os, mais qu'une implantation sous la peau soit toutefois possible.

2. Système de fixation selon la revendication 1, caractérisé en ce que chaque dispositif de serrage présente
- dans chaque alésage des sections finales (21) une partie de guidage (15) pour la tige (13) de la vis à os (10) avec une butée conique,
- un cône (16) à fente longitudinale, coulissant sur la tige (13) de la vis à os (10) et
- une tête plate (11) avec un alésage pour comprimer le cône à fente longitudinale (16) dans la butée conique.

3. Système de fixation selon la revendication 1 ou 2, caractérisé en ce que la partie de guidage (15) repose pour la majorité de sa surface avec sa base sur la partie finale (21) et est reliée à celle-ci par adhérence.

4. Système de fixation selon la revendication 1 ou 2, caractérisé en ce que la partie de guidage (15) est maintenue dans une section sphérique (17) qui repose par adhérence de façon pivotante dans un alésage de la section finale (21).

5. Système de fixation selon l'une des revendications précédentes caractérisé en ce que la surface de section limitée par les deux côtés étroits et par les deux côtés larges de la partie de liaison (31) de l'élément de fixation (20) est réduite - par rapport aux sections finales (21) - par diminution des dimensions (33) des côtés étroits ou des dimensions (32) des côtes larges ou des dimensions (33, 32) des côtés étroits et des côtés larges.

6. Système de fixation selon la revendication 5, caractérisé en ce que la réduction des dimensions (33) des côtés étroits de la partie de liaison (31) ou en ce que la réduction des mesures (32) des côtés larges de la partie de liaison (31 ), est de 30% à 70 %.

7. Système de fixation selon l'une des revendications précédentes, caractérisé en ce que l'élément de fixation (20) est composé de deux parties d'élément de fixation (25, 26) situées, à plat l'une sur l'autre qui sont fixées l'une contre l'autre (24) dans les sections finales.

8. Système de fixation selon la revendication 7, caractérisé en ce que les parties de fixation (35, 36) des parties d'élément de fixation (25, 26) peuvent être séparées l'une de l'autre par un système expansible (34, 37).

9. Système de fixation selon la revendication 8, caractérisé en ce que le système expansible est une vis sans tête (34) vissée dans un alésage fileté (37) dans la partie de liaison (35) de l'une des parties d'élément de fixation (25).

10. Système de fixation selon l'une des revendications 7 à 9 caractérisé en ce que les deux éléments de fixation (25, 26) sont d'épaisseur différente au moins dans leurs sections de liaison (35, 36) dans le sens de leur côté étroit.
